# EUROPÄISCHE PATENTSCHRIFT

(11) **EP 3 749 967 B1**
(45) Veröffentlichungstag und Bekanntmachung des Hinweises auf die Patenterteilung: **10.05.2023**
(21) Anmeldenummer: 19705762.3
(22) Anmeldetag: 16.02.2019
(51) Int. Cl.: G01R 19/22, G01R 31/42, A61B 18/12

(54) **PRÜFGERÄT FÜR EINEN ELEKTROCHIRURGISCHEN GENERATOR**
TESTING DEVICE FOR AN ELECTROSURGICAL GENERATOR
APPAREIL DE CONTRÔLE POUR UN GÉNÉRATEUR ÉLECTROCHIRURGICAL

(30) Priorität: 13.03.2018 DE 102018105824
(43) Veröffentlichungstag der Anmeldung: 16.12.2020
(73) Patentinhaber: OLYMPUS WINTER & IBE GMBH, 22045 Hamburg (DE)
(72) Erfinder: DIJKSTRA, Jelle, 10781 Berlin (DE)
(74) Vertreter: Noack, Andreas
(86) Internationale Anmeldenummer: PCT/EP2019/053907
(87) Internationale Veröffentlichungsnummer: WO 2019/174859

(56) Entgegenhaltungen:
- EP-A2- 0 391 233
- EP-A2- 0 750 382
- EP-B1- 2 468 359
- DE-A1-102004 010 769
- US-A- 6 142 992

## Beschreibung

Die Erfindung betrifft ein Prüfgerät für einen elektrochirurgischen Generator, mit einen ersten Anschlussport und einen zweiten Anschlussport zum Verbinden des Prüfgeräts mit einem Patientenstromkreis des elektrochirurgischen Generators, und mit einer Einrichtung zum Erzeugen einer Gleichspannung zwischen dem ersten Anschlussport und dem zweiten Anschlussport.

Elektrochirurgische Generatoren werden in der modernen Medizin für verschiedenste Zwecke eingesetzt. Dabei erzeugt der elektrochirurgische Generator zwischen zwei Anschlussports einen hochfrequenten Wechselstrom, oder HF-Strom, welcher mittels eines an den elektrochirurgischen Generator anzuschließenden Instruments in einem zu behandelnden Gewebe appliziert wird. Die Frequenz des HF-Stroms beträgt dabei zumeist zwischen 100kHz und 500kHz, es sind aber auch elektrochirurgische Generatoren bekannt, die im Frequenzbereich von bis zu 5MHz arbeiten.

Um ungewollte Stromflüsse sicher zu vermeiden wird der hochfrequente Wechselstrom in einem galvanisch getrennten Patientenstromkreis bereitgestellt.

Bei den anzuschließenden Instrumenten wird zwischen monopolaren und bipolaren Instrumenten unterschieden. Monopolare Instrumente sind mit nur einem Anschlussport des elektrochirurgischen Generators verbunden, hier wird der Patientenstromkreis über eine Applikationselektrode des Instruments und eine großflächig mit einer Körperoberfläche des Patienten verbundenen Neutralelektrode geschlossen, welche mit dem zweiten Anschlussport des elektrochirurgischen Generators verbunden ist. Bipolare Instrumente hingegen werden mit beiden Anschlussports des elektrochirurgischen Generators verbunden, sie weisen zwei Applikationselektroden auf.

Bei der Applikation des HF-Stroms kann es gewollt oder ungewollt zu einer Funkenbildung zwischen einer Applikationselektrode und dem zu behandelnden Gewebe bzw. einer weiteren Applikationselektrode kommen. Eine solche Funkenbildung muss von dem elektrochirurgischen Generator sicher erkannt werden.

Eine Funkenbildung führt zu einem unsymmetrischen Verlauf des Stromflusses durch den Patientenstromkreis. Dabei kann beispielsweise der Stromfluss in positiven Halbwellen des HF-Stroms gegenüber dem Stromfluss in negativen Halbwellen des HF-Stroms reduziert sein. Dieser unsymmetrische Verlauf des Stromflusses führt dazu, dass dem HF-Strom ein Gleichstromanteil überlagert wird, was jedoch aus Gründen der Patientensicherheit nicht zulässig ist.

Um diesen Gleichstromanteil zu unterdrücken, ist in den Patientenstromkreis ein Kondensator integriert. Über diesem Kondensator baut sich durch den unsymmetrischen Stromfluss eine Gleichspannung auf, sobald in dem Patientenstromkreis ein Funke zündet. Diese Gleichspannung wird durch die Steuerung des elektrochirurgischen Generators erkannt. Je nach gewünschten Behandlungseffekt kann die Steuerung den bereitgestellten HF-Strom dann so anpassen, dass der Funke entweder unterdrückt oder kontrolliert aufrechterhalten wird. In einzelnen Betriebsmodi eines elektrochirurgischen Generators kann auch bei Erkennung der Funkenbildung zwischen einzelnen Phasen einer Behandlung umgeschaltet werden.

Um die korrekte Funktion eines elektrochirurgischen Generators zu prüfen, muss dieser einem Funktionstest unterzogen werden. Dabei werden in allen zur Verfügung stehenden Betriebsmodi des elektrochirurgischen Generators die Kennwerte des ausgegebenen HF-Stroms ermittelt sowie die korrekte Abfolge der Behandlungsphasen überprüft.

Hierzu muss auch die korrekte Erkennung der Funkenbildung und die entsprechende Reaktion des elektrochirurgischen Generators geprüft werden. Eine tatsächliche Funkenbildung tritt aber nur in Zusammenspiel mit echtem Gewebe auf, und ist daher im Prüfablauf nur schwer zu realisieren.

Daher werden für die Überprüfung der Funkenerkennung von elektrochirurgischen Generatoren Prüfgeräte gattungsgemäße Prüfgeräte eingesetzt. Diese simulieren die Funkenentstehung dadurch, dass im Patientenstromkreis des elektrochirurgischen Generators eine Gleichspannung angelegt wird, welche in etwa der bei Funkenbildung entstehenden Gleichspannung entspricht.

Bekannte Prüfgeräte weisen dazu eine externe Stromversorgung auf, aus welcher die Einrichtung zur Erzeugung der Gleichspannung gespeist wird. Die Gleichspannung wird dabei in Serie zu einem HF-Strompfad angelegt, in welchem beispielsweise Messinstrumente für Stromstärke, Spannung und/oder Leistung des abgegebenen HF-Stroms angeordnet sind.

Dabei treten verschiedene Probleme auf. Zum einen kann die Erzeugung der Gleichspannung schwer mit der Abgabe des HF-Stroms durch den elektrochirurgischen Generator synchronisiert werden, z.B. bei Betriebsmodi mit schnell aufeinanderfolgenden Behandlungsphasen. Zum anderen wird die Komplexität des Prüfaufbaus durch die erforderliche Stromversorgung des Prüfgeräts weiter erhöht. Stand der Technik findet sich in den Dokumenten DE102004010769A1, EP0391233A2, US6142992A, EP2468359B1 , EP0750382A2.

Es besteht daher eine Aufgabe darin, ein Prüfgerät für elektrochirurgische Generatoren bereitzustellen, welches hinsichtlich der beschriebenen Problematik verbessert ist. Erfindungsgemäß wird diese Aufgabe gelöst durch ein Prüfgerät für einen elektrochirurgischen Generator, mit einen ersten Anschlussport und einen zweiten Anschlussport zum Verbinden des Prüfgeräts mit einem Patientenstromkreis des elektrochirurgischen Generators, und mit einer Einrichtung zum Erzeugen einer Gleichspannung zwischen dem ersten Anschlussport und dem zweiten Anschlussport, dadurch gekennzeichnet, dass ein HF-Strompfad des Patientenstromkreises von dem ersten Anschlussport zu dem zweiten Anschlussport durch das Prüfgerät verläuft, und dass die Einrichtung zum Erzeugen einer Gleichspannung durch einen durch den HF-Strompfad fließenden HF-Strom gespeist wird.

Durch diese Maßnahme ist die im Prüfgerät erzeugte Gleichspannung automatisch mit dem vom elektrochirurgischen Generator erzeugten HF-Strom synchronisiert, sie wird also genau dann aufgebaut, wenn der elektrochirurgische Generator HF-Strom abgibt. Gleichzeitig wird eine externe Stromversorgung für das Prüfgerät unnötig, dadurch vereinfacht sich der Prüfaufbau erheblich.

In einer möglichen Ausführung der Erfindung kann dabei die Einrichtung zum Erzeugen einer Gleichspannung einen Kondensator umfassen, welcher in dem HF-Strompfad angeordnet ist. Dabei kann die Gleichspannung durch das Aufladen des Kondensators erzeugt werden, wobei gleichzeitig der HF-Strom bei angemessener Dimensionierung des Kondensators kaum beeinflusst wird.

Gemäß einer Weiterbildung der Erfindung kann die Einrichtung zum Erzeugen einer Gleichspannung eingerichtet sein, positive Halbwellen des HF-Stroms durch den Kondensator zu leiten, und negative Halbwellen des HF-Stroms nicht durch den Kondensator zu leiten. Somit wird der Kondensator durch die positiven Halbwellen des HF-Stroms aufgeladen, ohne dass er durch die negativen Halbwellen wieder entladen wird.

Die Bezeichnungen "positive Halbwelle" und "negative Halbwelle" tragen dabei der Tatsache Rechnung, dass sich bei einem Wechselstrom die Stromrichtung periodisch ändert. Dabei wird der Abschnitt des Stromverlaufs, bei dem der Strom in eine erste Richtung fließt, als "positive Halbwelle" bezeichnet, und der Abschnitt, in dem der Strom in die entgegengesetzte Richtung fließt, als "negative Halbwelle". Dabei ist die Auswahl, welcher Abschnitt als positive Halbwelle bezeichnet wird, willkürlich. Der Begriff "Halbwelle" wird dabei auch bei unsymmetrischen Wechselströmen benutzt und impliziert nicht, dass entgegengesetzte Halbwellen sich in bestimmten Parametern wir Zeitdauer, Amplitude, Signalform o.ä. entsprechen.

In einer bevorzugten Umsetzung eines Prüfgeräts nach der Erfindung kann die Einrichtung zum Erzeugen einer Gleichspannung zwei parallele HF-Strompfade aufweisen, wobei ein erster HF- Strompfad den Kondensator umfasst und eine erste Diode aufweist, die in Richtung der positiven Halbwellen des HF-Stroms gepolt ist, und wobei ein zweiter HF-Strompfad eine zweite Diode aufweist, die in Richtung der negativen Halbwellen des HF-Stroms gepolt ist. Auf diese Weise lässt sich die Trennung der Halbwellen des HF-Stroms besonders einfach erreichen.

Ein Prüfgerät nach der Erfindung kann dabei im ersten HF-Strompfad einen Schalter zum Überbrücken der ersten Diode aufweisen. Durch Schließen des Schalters wird die gleichrichtende Wirkung der ersten Diode aufgehoben, so dass beide Halbwellen des HF-Stroms durch den Kondensator fließen können und dieser sich somit nicht weiter auflädt.

Als Schalter im Sinne der Erfindung kommen alle elektrisch steuerbaren Schalter in Frage, wie z.B. Relais. Vorzugsweise werden hier jedoch Transistoren eingesetzt, z.B. MOSFETs.

Gemäß einer besonders bevorzugten Ausführung eines Prüfgeräts nach der Erfindung ist dabei die Einrichtung zum Erzeugen einer Gleichspannung eingerichtet, den ersten Schalter zu schließen, wenn die Gleichspannung über dem Kondensator einen vorgegebenen oder vorgebbaren Wert erreicht. Auf diese Weise kann die erzeugte Gleichspannung auf einen Wert begrenzt werden, welcher üblicherweise bei einer Funkenbildung auftreten würde. Dieser Wert kann beispielsweise zwischen 5V und 300V, vorzugsweise zwischen 50V und 200V, bevorzugt zwischen 80V und 170V, und besonders bevorzugt zwischen 100V und 150V liegen.

In einer weiteren möglichen Ausgestaltung der Erfindung kann das Prüfgerät eine Einrichtung zum Entladen des Kondensators aufweisen. Mittels einer solchen Einrichtung kann die Gleichspannung durch Entladen des Kondensators schnell reduziert bzw. vollständig abgebaut werden.

Vorteilhafterweise kann die Einrichtung zum Entladen des Kondensators eingerichtet sein, den Kondensator zu entladen, wenn kein HF-Strom durch den HF-Strompfad fließt. Hierdurch kann das Löschen eines Funkens durch Abschaltung des HF-Stroms simuliert werden.

Die Einrichtung zum Entladen des Kondensators kann in einer bevorzugten Ausführung der Erfindung einen zweiten Schalter umfassen. Hierbei kann es sich ebenfalls um einen MOSFET handeln.

Die Erfindung wird nachfolgend anhand einiger exemplarischer Darstellungen näher erläutert. Es zeigen:
Fig. 1: Einen elektrochirurgischen Generator mit angeschlossenem Prüfgerät gemäß Stand der Technik,
Fig. 2: einen elektrochirurgischen Generator mit angeschlossenem Prüfgerät gemäß der Erfindung,
Fig. 3: den prinzipiellen Aufbau eines Prüfgeräts gemäß der Erfindung.

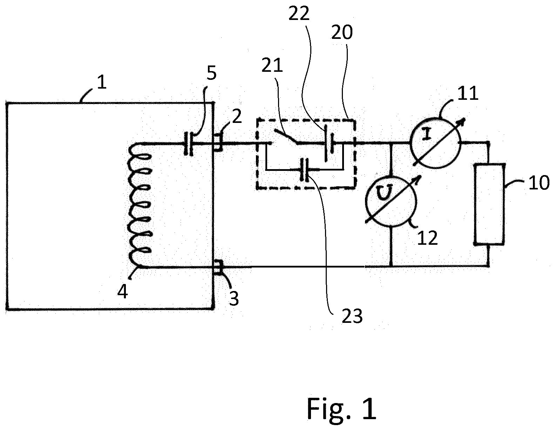

In Figur 1 ist ein elektrochirurgischer Generator 1 dargestellt. Der elektrochirurgische Generator 1 umfasst Anschlussports 2,3, die Bestandteil eines Patientenstromkreises sind. Der Patientenstromkreis ist galvanisch von der nicht dargestellten Stromversorgung des elektrochirurgischen Generators 1 getrennt, dies ist durch eine Transformatorspule 4 angedeutet. Ein Kondensator 5 unterdrückt Gleichstromanteile im Patientenstromkreis.

Bei normalem Betrieb des elektrochirurgischen Generators 1 kann ein nicht dargestelltes elektrochirurgisches Instrument und ggf. eine Neutralelektrode an die Anschlussports 2,3 angeschlossen werden.

Figur 1 zeigt den elektrochirurgischen Generator 1 in einer Testkonfiguration. Um die korrekte Funktionsweise des elektrochirurgischen Generators 1 zu testen, ist an die Anschlussports 2,3 ein Teststromkreis mit einem Lastwiderstand 10 angeschlossen. Der vom elektrochirurgischen Generator 1 abgegebene HF-Strom sowie die über dem Lastwiderstand 10 abfallende Spannung werden durch die Messgeräte 11,12 gemessen und protokolliert.

Um eine bei Funkenentstehung anfallende Gleichspannung im Patientenstromkreis zu simulieren, ist in den Teststromkreis ein Prüfgerät 20 integriert. Das Prüfgerät 20 umfasst eine Gleichspannungsquelle 21 sowie einen Schalter 22, über welche die Gleichspannung zu- und abgeschaltet werden kann. Parallel zu der Gleichspannungsquelle 21 und dem Schalter 22 ist ein Kondensator 23 geschaltet, um den vom Generator 1 abgegebenen HF-Strom durch das Prüfgerät 20 zu leiten.

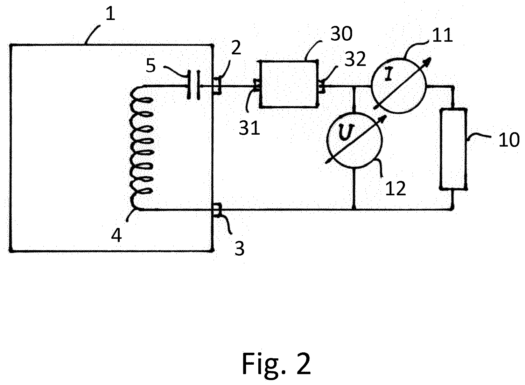

In Figur 2 ist wiederum der elektrochirurgische Generator 1 dargestellt, der an einen Teststromkreis mit einem Lastwiderstand 10 angeschlossen ist. Ebenfalls sind die Messgeräte 11,12 dargestellt.

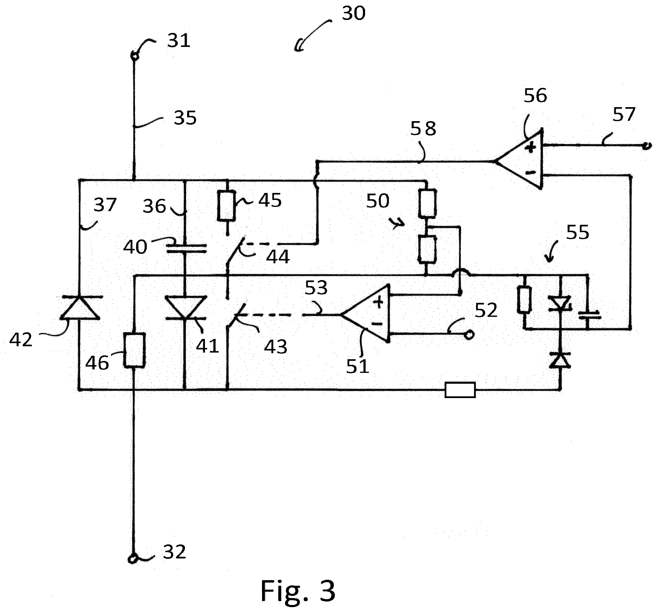

Anstelle des Prüfgeräts 20 ist in den Patientenstromkreis des elektrochirurgischen Generators 1 ein Prüfgerät 30 mit Anschlussports 31,32 integriert, welches gemäß der Erfindung ausgeführt ist. Der innere Aufbau des Prüfgeräts 30 ist in Figur 3 dargestellt.

Figur 3 zeigt den prinzipiellen elektronischen Aufbau des Prüfgeräts 30. Zwischen den Anschlussports 31,32 erstreckt sich ein HF-Strompfad 35. Der HF-Strompfad verzweigt sich in zwei parallele HF-Strompfade 36,37. Der erste HF-Strompfad 36 umfasst einen Kondensator 40 und eine erste Diode 41, der zweite Strompfad 37 umfasst eine zweite Diode 42.

Die Dioden 41,42 sind entgegengesetzt gepolt, so dass positive Halbwellen eines durch das Prüfgerät 30 fließenden HF-Stroms durch den ersten Strompfad 36 mit dem Kondensator 40 fließen müssen und dabei den Kondensator 40 aufladen. Negative Halbwellen des HF-Stroms fließen hingegen durch den zweiten Strompfad 37.

Infolge dieses Aufbaus ist der durch den Kondensator 40 fließende HF-Strom stark unsymmetrisch, so dass sich über dem Kondensator eine Gleichspannung aufbaut. Diese Gleichspannung liegt auch zwischen den Anschlussports 31,32 an und ist somit für den elektrochirurgischen Generator 1 sichtbar.

Sobald die Gleichspannung über dem Kondensator 40 einen vorgegebenen oder vorgebbaren Wert erreicht hat, kann durch Schließen eines Schalters 43 die Diode 41 überbrückt werden. Es können somit beide Halbwellen des HF-Stroms durch den HF-Strompfad 37 fließen, und der Kondensator 40 wird nicht weiter aufgeladen.

Ein zweiter Schalter 44 dient dazu, den Kondensator 40 über einen Widerstand 45 kontrolliert zu entladen. Dabei ist der Widerstand 45 so dimensioniert, dass eine Entladung des Kondensators 40 in einigen Millisekunden erfolgt.

Parallel zu der Diode 41 ist ein weiterer Widerstand 46 geschaltet, welcher dazu dient, bei gepulsten Betriebsmodi des elektrochirurgischen Generators 1 in den Pulspausen einen Potentialausgleich mit dem Kondensator 5 im elektrochirurgischen Generator 1 zu ermöglichen. Dabei hat das RC-Glied aus Kondensator 40 und Widerstand 46 eine so große Zeitkonstante, dass es für den HF-Strom unbedeutend ist.

Wie bereits angedeutet erfolgt die Steuerung der im Prüfgerät 30 erzeugten Gleichspannung mittels des Schalters 43, dieser ist als MOSFET realisiert. Die über dem Kondensator 40 anliegende Gleichspannung wird über einen Spannungsteiler 50 auf den positiven Eingang eines Komparators 51 gegeben. An den negativen Eingang des Komparators 51 ist eine feste Referenzspannung 52 angelegt. Der Ausgang 53 des Komparators 51 ist mit dem Gate des Schalters 43 verbunden.

Erreicht nun die Ausgangsspannung des Spannungsteilers 50 die Referenzspannung 52, so schaltet der Komparator 51, und der Schalter 43 wird geschlossen. Damit wird der Kondensator 40 nicht weiter aufgeladen.

Da sich der Kondensator 40 über den Widerstand 46 und den Spannungsteiler 50 langsam entlädt, wird die Ausgangsspannung des Spannungsteilers 50 die Referenzspannung 52 bald wieder unterschreiten, so dass der Schalter 43 wieder geöffnet wird. Um ein zu schnelles Umschalten des Schalters 43 zu vermeiden, kann der Komparator 51 mit einer Hystereseschaltung versehen werden.

Die Diode 41 kann Bestandteil des MOSFET sein, welcher den Schalter 43 bildet.

Der Schalter 44 zum Entladen des Kondensators 40 ist ebenfalls als MOSFET ausgeführt und wird auf ähnliche Art gesteuert wie der Schalter 43. Dazu wird die über der Diode 41 anliegende Spannung über einen Diodengleichrichter 55 gleichgerichtet und auf den negativen Eingang eines Komparators 56 gelegt. Auf den positiven Eingang des Komparators 56 wird eine feste Referenzspannung 57 gelegt. Der Ausgang 58 des Komparators 56 ist mit dem Gate des Schalters 44 verbunden.

Solange HF-Strom durch das Prüfgerät fließt, ist die Ausgangsspannung des Diodengleichrichters 55 höher als die Referenzspannung 57. Schaltet jetzt der elektrochirurgische Generator den HF-Strom ab, beispielsweise um einen ungewollten Funken zu löschen, oder in einer Pulspause in einem gepulsten Betriebsmodus, so fällt die Ausgangsspannung des Diodengleichrichters 55 ab und der Komparator 56 schaltet. Dadurch wird der Schalter 44 geschlossen und der Kondensator 40 entlädt sich schnell über den Widerstand 45.

Eine Betriebsspannung für die Komparatoren und die MOSFETS kann mittels eines nicht dargestellten DC-DC-Wandlers aus der Spannung über dem Kondensator 40 gewonnen werden.

## Patentansprüche

1. Prüfgerät für einen elektrochirurgischen Generator (1), mit einen ersten Anschlussport (2) und einen zweiten Anschlussport (3) zum Verbinden des Prüfgeräts mit einem Patientenstromkreis des elektrochirurgischen Generators (1), und mit einer Einrichtung zum Erzeugen einer Gleichspannung zwischen dem ersten Anschlussport (2) und dem zweiten Anschlussport (3),
**dadurch gekennzeichnet, dass** ein HF-Strompfad (35,36,37) des Patientenstromkreises von dem ersten Anschlussport (2) zu dem zweiten Anschlussport (3) durch das Prüfgerät verläuft, und dass die Einrichtung zum Erzeugen einer Gleichspannung durch einen durch den HF-Strompfad (35,36,37) fließenden HF-Strom gespeist wird.

2. Prüfgerät nach Anspruch 1, **dadurch gekennzeichnet, dass** die Einrichtung zum Erzeugen einer Gleichspannung einen in dem HF-Strompfad (35,36) angeordneten Kondensator (40) umfasst.

3. Prüfgerät nach Anspruch 2, **dadurch gekennzeichnet, dass** die Einrichtung zum Erzeugen einer Gleichspannung eingerichtet ist, positive Halbwellen des HF-Stroms durch den Kondensator (40) zu leiten, und negative Halbwellen des HF-Stroms nicht durch den Kondensator zu leiten.

4. Prüfgerät nach Anspruch 3, **dadurch gekennzeichnet, dass** die Einrichtung zum Erzeugen einer Gleichspannung zwei parallele HF-Strompfade (36,37) aufweist, wobei ein erster HF- Strompfad (36) den Kondensator (40) umfasst und eine erste Diode (41) aufweist, die in Richtung der positiven Halbwellen des HF-Stroms gepolt ist, und wobei ein zweiter HF-Strompfad (37) eine zweite Diode (42) aufweist, die in Richtung der negativen Halbwellen des HF-Stroms gepolt ist.

5. Prüfgerät nach Anspruch 4, **dadurch gekennzeichnet, dass** der erste HF-Strompfad (36) einen ersten Schalter (43) zum Überbrücken der ersten Diode (41) aufweist.

6. Prüfgerät nach Anspruch 5, **dadurch gekennzeichnet, dass** die Einrichtung zum Erzeugen einer Gleichspannung eingerichtet ist, den ersten Schalter (43) zu schließen, wenn die Gleichspannung über dem Kondensator (40) einen vorgegebenen oder vorgebbaren Wert erreicht.

7. Prüfgerät nach einem der Ansprüche 2 bis 6, **dadurch gekennzeichnet, dass** das Prüfgerät eine Einrichtung (44,45) zum Entladen des Kondensators (40) aufweist.

8. Prüfgerät nach Anspruch 7, **dadurch gekennzeichnet, dass** die Einrichtung (44,45) zum Entladen des Kondensators (40) eingerichtet ist, den Kondensator (40) zu entladen, wenn kein HF-Strom durch den HF-Strompfad (35,36,37) fließt.

9. Prüfgerät nach Anspruch 8, **dadurch gekennzeichnet, dass** die Einrichtung (44,45) zum Entladen des Kondensators (40) einen zweiten Schalter (44) umfasst.

## Claims

1. Test apparatus for an electrosurgical generator (1), having a first connection port (2) and a second connection port (3) for connecting the test apparatus to a patient circuit of the electrosurgical generator (1), and having means for generating a DC voltage between the first connection port (2) and the second connection port (3),
**characterized in that** an RF current path (35,36,37) of the patient circuit extends from the first connection port (2) to the second connection port (3) through the test apparatus, and **in that** the means for generating a DC voltage is powered by an RF current flowing through the RF current path (35,36,37).

2. Test apparatus according to claim 1, **characterized in that** the means for generating a DC voltage comprises a capacitor (40) arranged in the RF current path (35,36).

3. Test apparatus according to claim 2, **characterized in that** the means for generating a DC voltage is configured to pass positive half-waves of the RF current through the capacitor (40) and not to pass negative half-waves of the RF current through the capacitor.

4. Test apparatus according to claim 3, **characterized in that** the means for generating a DC voltage comprises two parallel RF current paths (36,37), a first RF current path (36) comprising the capacitor (40) and having a first diode (41) poled in the direction of the positive half-waves of the RF current, and a second RF current path (37) having a second diode (42) poled in the direction of the negative half-waves of the RF current.

5. Test apparatus according to claim 4, **characterized in that** the first RF current path (36) comprises a first switch (43) for bypassing the first diode (41).

6. Test apparatus according to claim 5, **characterized in that** the means for generating a DC voltage is configured to close the first switch (43) when the DC voltage across the capacitor (40) reaches a predetermined or predeterminable value.

7. Test apparatus according to any one of claims 2 to 6, **characterized in that** the test apparatus comprises means (44, 45) for discharging the capacitor (40).

8. Test apparatus according to claim 7, **characterized in that** the means (44,45) for discharging the capacitor (40) is configured to discharge the capacitor (40) when no RF current is flowing through the RF current path (35,36,37).

9. Test apparatus according to claim 8, **characterized in that** the means (44,45) for discharging the capacitor (40) comprises a second switch (44).

## Revendications

1. Appareil de test pour un générateur électrochirurgical (1), comprenant un premier port de connexion (2) et un deuxième port de connexion (3) pour relier l'appareil de test à un circuit de patient du générateur électrochirurgical (1), et comprenant un moyen pour générer une tension continue entre le premier port de connexion (2) et le deuxième port de connexion (3),
**caractérisé en ce qu'**un trajet de courant RF (35, 36, 37) du circuit patient s'étend du premier port de connexion (2) au deuxième port de connexion (3) à travers l'appareil de test, et **en ce que** le moyen pour générer une tension continue est alimenté par un courant RF s'écoulant à travers le trajet de courant RF (35, 36, 37).

2. Appareil de test selon la revendication 1, **caractérisé en ce que** le moyen pour générer une tension continue comprend un condensateur (40) disposé dans le trajet de courant RF (35, 36).

3. Appareil de test selon la revendication 2, **caractérisé en ce que** le moyen pour générer une tension continue est adapté pour faire passer des demi-ondes positives du courant RF à travers le condensateur (40) et pour ne pas faire passer des demi-ondes négatives du courant RF à travers le condensateur.

4. Appareil de test selon la revendication 3, **caractérisé en ce que** le moyen pour générer une tension continue comprend deux trajets de courant RF parallèles (36, 37), un premier trajet de courant RF (36) comprenant le condensateur (40) et comprenant une première diode (41) polarisée dans le sens des demi-ondes positives du courant RF, et un second trajet de courant RF (37) comprenant une seconde diode (42) polarisée dans le sens des demi-ondes négatives du courant RF.

5. Appareil de test selon la revendication 4, **caractérisé en ce que** le premier trajet de courant RF (36) comprend un premier commutateur (43) pour ponter la première diode (41).

6. Appareil de test selon la revendication 5, **caractérisé en ce que** le moyen pour générer une tension continue est configuré pour fermer le premier commutateur (43) lorsque la tension continue à travers le condensateur (40) atteint une valeur prédéterminée ou prédéterminable.

7. Appareil de test selon l'une quelconque des revendications 2 à 6, **caractérisé en ce que** l'appareil de test comprend un moyen (44, 45) pour décharger le condensateur (40).

8. Appareil de test selon la revendication 7, **caractérisé en ce que** le moyen (44, 45) pour décharger le condensateur (40) est configuré pour décharger le condensateur (40) lorsqu'aucun courant RF ne circule dans le trajet de courant RF (35, 36, 37).

9. Appareil de test selon la revendication 8, **caractérisé en ce que** le moyen (44, 45) pour décharger le condensateur (40) comprend un deuxième commutateur (44).
